# EUROPEAN PATENT SPECIFICATION

(11) **EP 1 658 281 B1**
(45) Date of publication and mention of the grant of the patent: **29.09.2010**
(21) Application number: 04762303.8
(22) Date of filing: 26.08.2004
(51) Int. Cl.: C07D 403/10, C07D 257/04

(54) **A METHOD OF REMOVING THE TRIPHENYLMETHANE PROTECTING GROUP**
VERFAHREN ZUR ENTFERNUNG DER TRIPHENYLMETHANSCHUTZGRUPPE
PROCEDE D'EXTRACTION DU GROUPE PROTECTEUR DE TRIPHENYLMETHANE

(30) Priority: 27.08.2003 CZ 20032319; 16.06.2004 CZ 20040733
(43) Date of publication of application: 24.05.2006
(73) Proprietor: Zentiva, k.s., Dolni Mecholupy 102 37 Praha 10 (CZ)
(72) Inventor: RADL, Stanislav, 25084 Kvetnice (CZ); STACH, Jan, 190 16 Praha 9 - Ujezd nad Lesy (CZ); KLECAN, Ondrej, 397 01 Pisek (CZ)
(74) Representative: Jirotkova, Ivana
(86) International application number: PCT/CZ2004/000051
(87) International publication number: WO 2005/021535

(56) References cited:
- EP-A- 0 497 121
- WO-A-02/094816
- US-A- 5 196 444
- US-A- 5 281 604
- US-A- 5 399 578
- US-A- 5 559 233
- NICHOLAS, J. S. HARMAT ET AL: "4-Diazinyl- and 4-Pyridinylimidazoles: Potent Angiotensin II Antagonists. A Study of Their Activity and Computational Characterization" JOURNAL OF MEDICINAL CHEMISTRY, vol. 38, no. 15, 1995, pages 2925-2937, XP002313313
- CARINI D J ET AL: "NONPEPTIDE ANGIOTENSIN II RECEPTOR ANTAGONISTS: THE DISCOVERY OF A SERIES OF N-(BIPHENYLYLMETHYL)IMIDAZOLES AS POTENT, ORALLY ACTIVE ANTIHYPERTENSIVES" JOURNAL OF MEDICINAL CHEMISTRY, AMERICAN CHEMICAL SOCIETY. WASHINGTON, US, vol. 34, no. 8, 1 August 1991 (1991-08-01), pages 2525-2547, XP000674205 ISSN: 0022-2623
- USIFOH, CYRIL O.: "Anticonvulsant activity of reaction products of 5,5-diphenylhydantoin with substituted methylene bromides" ARCH. PHARM. PHARM. MED. CHEM., vol. 334, no. 11, 2001, pages 366-368, XP002319792
- ATTANASI O. A. ET AL.: "Synthesis of biphenyltetrazole derivatives of 1-aminopyrroles as angiotensin II antagonists" FARMACO, vol. 54, 1999, pages 64-76, XP002319793

## Description

This invention relates to an improved method of removing the triphenylmethane (trityl) protecting group from trityl losartan of formula IV 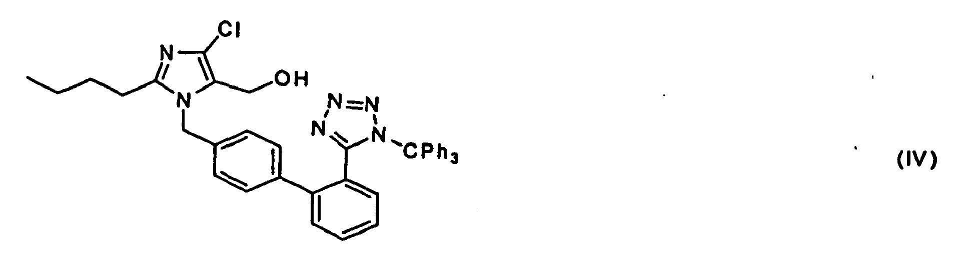 and a method of its use for the production of a drug for regulation of blood pressure from the antagonist of angiotensin II of formula III

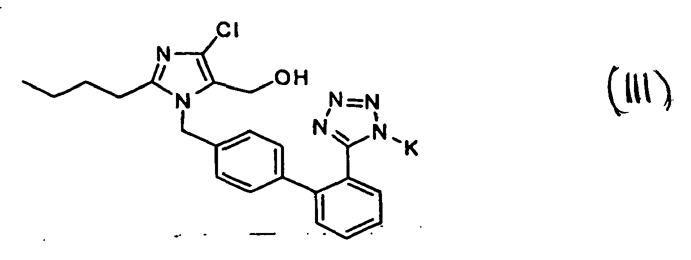

### Background Art

The potassium salt of losartan of formula III 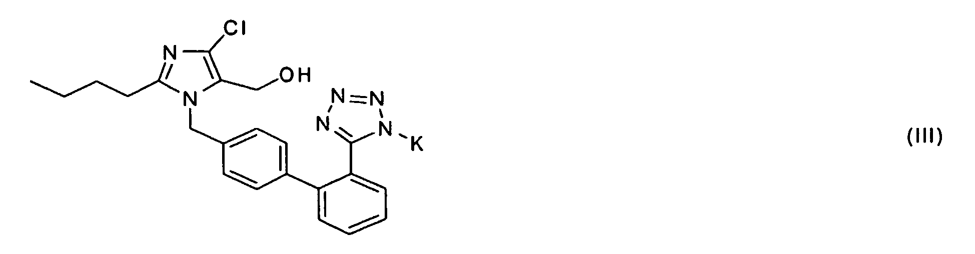 is produced according to published processes (WO 95/17396, EP 253310, US 5,859,258; J. Med. Chem. 1991, 34, 2525; J. Org. Chem. 1994, 59, 6391) by several methods which use trityl losartan of formula IV as a key intermediate.

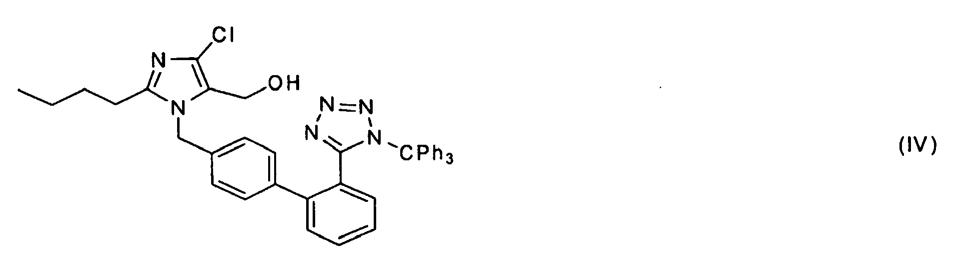

According to the original patents, trityl losartan of formula IV was transformed by acid hydrolysis to 2-butyl-4-chloro-1-[[(2'-tetrazol-5-yl)-1,1'-biphenyl-4-yl]methyl]-5-hydroxymethyl-imidazole, hereinafter referred to as "losartan acid" of formula V 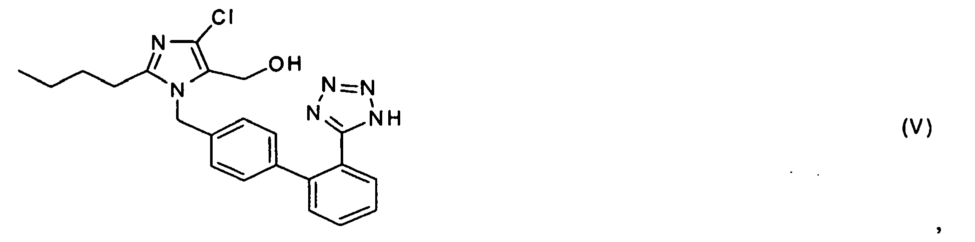 which was isolated and then transformed by potassium hydroxide to the potassium salt of losartan of formula III.

When removing the trityl protective group, strongly corrosive acids are usually used. The need of isolation of the free acid of losartan and a complicated removal of excess mineral acids from the product are disadvantages of this method. The free acid prepared in this way is then transformed by aqueous potassium hydroxide to the potassium salt, which is then, according to the above-mentioned patents, dissolved in isopropanol and the product crystallizes after azeotropic distillation with cyclohexane. Especially the lengthy azeotropic distillation is a disadvantage here.

On the basis of more recent patent applications (WO 01/61336; WO 02/094816), the trityl protecting group can also be removed by the action of strongly alkaline potassium hydroxide in primary alcohols. The potassium salt of losartan of formula III can be prepared by this method and the subsequent crystallization is carried out by adding a solvent in which the potassium salt of losartan is insoluble. However, during the said alkaline detritylation by a strong base, some minor impurities are formed and it is difficult to remove them from the product.

One of the best possibilities how to synthesize irbesartan of formula VI 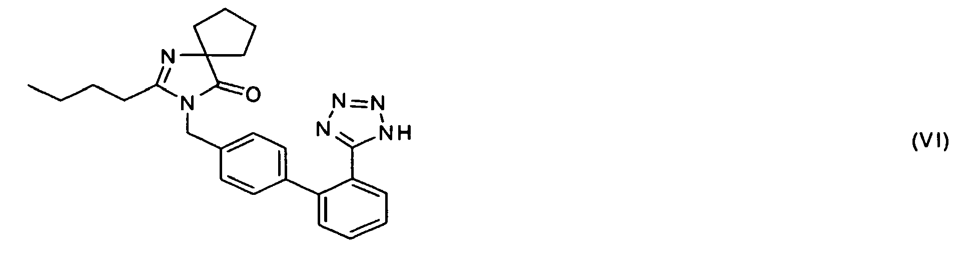 is synthesis via trityl irbesartan of formula VII 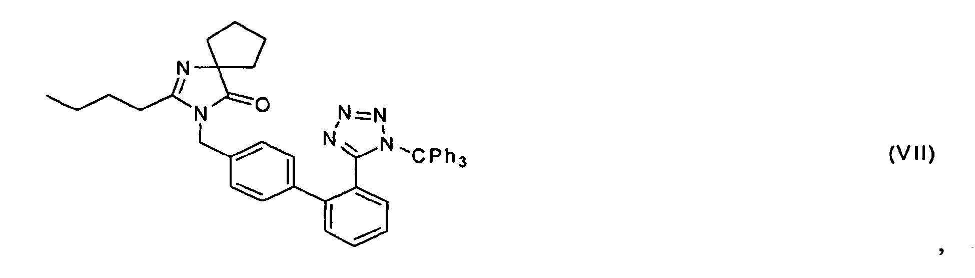 described in patent (US 5,559,233).

By removing the trityl protecting group, directly irbesartan of formula VI is obtained. The above-mentioned patent also uses detritylation in an acid medium, which has the already-discussed disadvantages.

The key intermediate of one of the most advantageous syntheses of valsartan of formula VIII 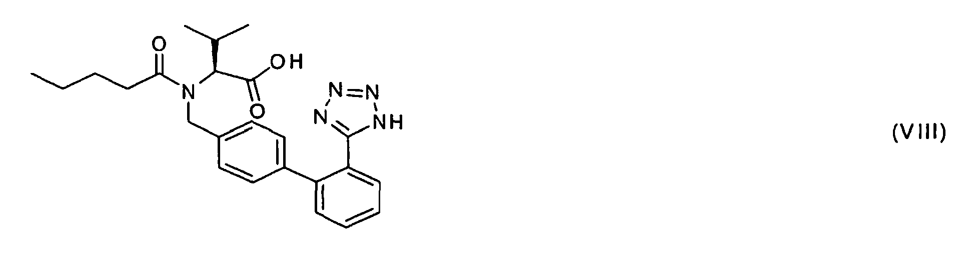 is the benzyl ester of trityl valsartan of formula IX

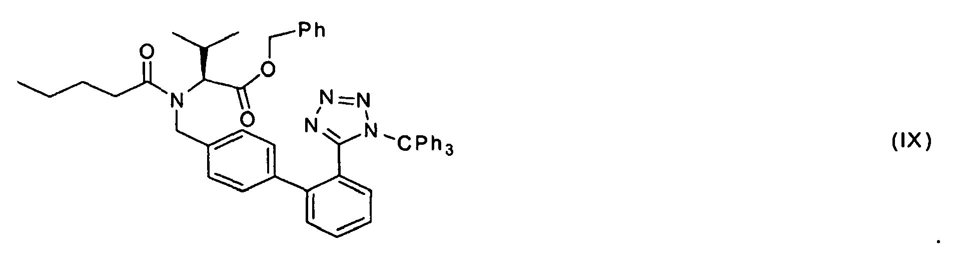

Valsartan of formula VIII is obtained according to the published patent (US 5,399,578) in such a way that the benzyl ester of trityl valsartan of formula IX is detritylated by the action of hydrochloric acid in dioxane, thus giving the benzyl ester of valsartan of formula X 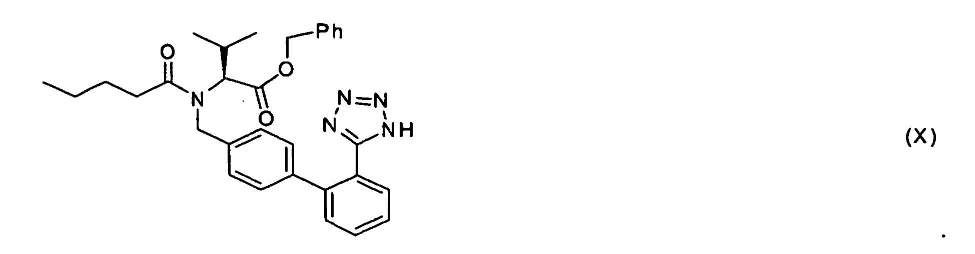 In a second step, the benzyl ester protecting group is removed by catalytic hydrogenation and valsartan of formula VIII is obtained.

A different method was used for isotope-labelled valsartan, wherein both protecting groups were removed by catalytic hydrogenation (J. Labelled. Cpd. Radiopharm. 2000, 43, 1245). A disadvantage of the first process is the use of corrosive hydrochloric acid. In the catalytic hydrogenation of both protecting groups, again, the use of a catalyst containing palladium increases costs. In both cases, triphenylmethanol or triphenylmethane, which are formed during the reactions, have to be removed by complicated extractions.

Besides the above-mentioned methods of detritylation, also detritylation catalyzed by anhydrous acids in anhydrous alcohols, preferably in methanol, is described for similar substances of the sartan type (US 5,763,619). According to the information in the said patent, an advantage of this method is that no splitting off of other hydrolysable functions occurs.

Candesartan cilexetil is produced according to published patents (US patent 5,196,444 and US patent 5,763,619) using the following method:

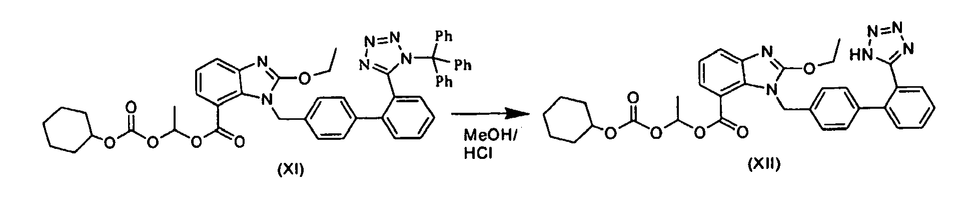

The synthesis starts with 1-(cyclohexyloxycarbonyloxy)ethyl-2-ethoxy-1-[[2'-(*N-*triphenylmethyl-1*H*-tetrazol-5-yl)biphenyl-4-yl]methyl]benzimidazol-7-carboxylate of formula XI, which is, in methanol by means of hydrochloric acid, transformed to candesartan cilexetil of formula XII. Synthesis of the starting substance XI is described in the original patent (US patent 5,196,444) and the compound is nowadays commercially available. The method of detritylation described in the original patent (US patent 5,196,444) has a very low yield and the product has to be purified chromatographically. The Takeda company improved this key step by using anhydrous hydrogen chloride in methanol (US patent 5,763,619), wherein the proportion of the decomposition products is lower and the yield higher.

In US 5,763,619, this method is not used for detritylation of any intermediate useful for the production of losartan, irbesartan or valsartan. At least in the case of valsartan, partial reesterification and, probably, partial splitting off of the valeroyl residue would presumably occur. Similarly, cleavage of the dihydroimidazolone ring could also be expected in the case of irbesartan. Another disadvantages seem to be fluctuation of yields (in the examples they fluctuate from 42 % to 92 %), corrosiveness of the reaction medium, and the need to use water when removing the excess of the acid used, which partially eliminates the advantages of reaction in an anhydrous medium. Moreover, in the case of drugs used in the form of alkali salts (for example losartan), it is then necessary to transform the isolated "acid" to the respective salt. In view of the fact that the best used acid is a solution of anhydrous hydrogen chloride in an anhydrous alcohol, the need to prepare an anhydrous solution of the acid used in the respective alcohol is also an important disadvantage.

Drawbacks of the above-mentioned methods include the use of strongly corrosive acids and also the need to process the reaction mixture by complex extractions. Such a production is then economically disadvantageous.

### Disclosure of the Invention

The object of the invention is an improved method of removing the triphenylmethane (trityl) protecting group from 1-triphenylmethyl-5-(4'-subst. aminomethyl-1,1'-biphenyl-2-yl)-1*H*-tetrazoles and a method of its use for the production of the potassium salt of 2-butyl-4-chloro-1-[[(2'-1*H*-tetrazol-5-yl)-1,1'-biphenyl-4-yl]methyl]-5-hydroxymethyl-imidazole (losartan) of formula III.

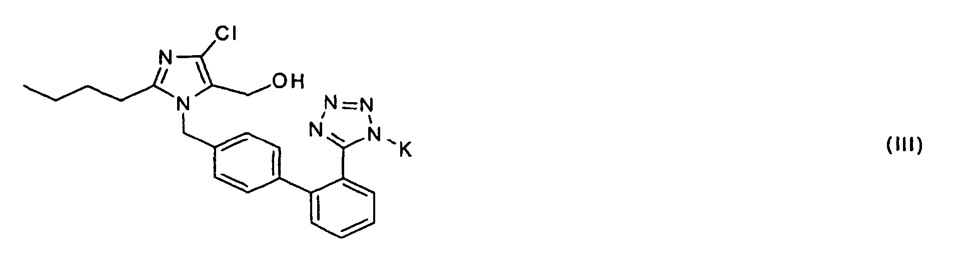

The said drug, which is a therapeutically important remedy used for regulation of blood pressure, belongs to a medicine group called antagonists of angiotensin 11 receptor.

This whole method is based on the surprising discovery that the removal of the trityl protecting group from the compound of formula III can be carried out by solvolysis under reflux in anhydrous methanol, without the presence of any acidic or basic agents.

The "losartan acid" of formula V, obtained in this way from trityl losartan of formula IV, is then transformed by the action of the weak bases potassium hydrogencarbonate or potassium carbonate, to the potassium salt of losartan of formula III. The transformation of trityl losartan of formula IV to the potassium salt of losartan of formula III can also be carried out by adding the said weak base at the beginning of the reaction.

A detailed description of the invention follows;

Detritylation in methanol alone without adding any catalyst proceeds by stirring the respective tritylated intermediate with methanol at temperatures between 20 °C and the boiling point of methanol, advantageously under reflux, when the reaction is completed within several hours.

If trityl losartan of formula IV is the starting tritylated intermediate, a solution of free "losartan acid" of formula V is obtained by the said method and is then transformed to the potassium salt of losartan of formula III by the action potassium carbonate or potassium hydrogencarbonate. The crystallization itself can then be carried out from mixtures of an alcohol, advantageously isopropanol, and an antisolvent, in which the potassium salt of losartan of formula III is insoluble, or with the use of other solvents, for example acetone. When using this method, an enormously pure product can be obtained, not containing impurities which are usual for the acid method, or for the method using potassium hydroxide. Deprotection can be, without a substantial deterioration of the purity of the crude potassium salt of losartan of formula III, also carried out directly in the presence of the weak base potassium carbonate or hydrogencarbonate, wherein directly the said potassium salt of losartan of formula III is the product.

The invention is elucidated in greater detail in the following working examples. These examples, which illustrate improvement of the method of the invention, are of an illustrative nature only and do not limit the scope of the invention in any way.

### Examples

### Example 1

### 2-Butyl-4-chloro-1-[[(2'-tetrazol-5-yl)-1,1'-biphenyl-4-yl]methyl]-5-hydroxymethyl-imidazole

A suspension of 10 g (0.015 mol) of 2-butyl-4-chloro-1-[[(2'-1-trityl-1*H*-tetrazol-5-yl)-1,1-biphenyl-4-yl]methyl]-5-hydroxymethyl-imidazole (trityl losartan, IV) in 50 ml of anhydrous methanol was refluxed for 7 hours. The solution was then cooled to -10 °C and stirred at this temperature overnight, the precipitated crystals were sucked off and washed with a small amount of ice-cold methanol. 3.7 g (90 %) of methyltriphenylmethyl ether (XIII) were obtained. The combined mother liquors were evaporated and boiled with 50 ml of hexane, the mixture was cooled and the insoluble part was sucked off, stirred at room temperature with 50 ml of cyclohexane for 10 hr, the insoluble part was sucked off. 6.2 g of the product (98 %) were obtained with mp of 186-188 °C. ¹H NMR spectra (DMSO): 0.81 t, *J* = 7.24, 3H; 1.27 m, 2H; 1.47 m, 2H; 2.47 t, *J* = 7.57, 2H; 4.35 s, 2H; 5.26 s, 2H; 7.03-7.12 m, 4H; 7.49-7.73 m, 4H.

### Example 2

### Potassium salt of 2-butyl-4-chloro-1-[(2'-tetrazol-5-yl)-1,1'-biphenyl-4-yl]-5-(hydroxymethyl)-imidazole (losartan, III)

A suspension of 10 g (0.015 mol) of 2-butyl-4-chloro-1-[(2'-1-trityl-1*H*-tetrazol-5-yl)-1,1-biphenyl-4-yl]-5-(hydroxymethyl)-imidazole (trityl losartan, IV) in 100 ml of anhydrous methanol was refluxed for 7 hr. The solution was then concentrated to ca 1/5 of its volume and the after-cooling-precipitated methyltriphenylmethyl ether (XIII) was sucked off and washed with a small amount of ice-cold methanol. 3.71 g (90 %) of methyltriphenylmethyl ether (XIII) were obtained. The filtrate was evaporated and the evaporation residue was dissolved in 100 ml of methanol. 1.50 g of KHCO₃ was added and the mixture was refluxed for 4 hr. Methanol was then evaporated and after acetone was added the evaporation residue crystallized. The crystals were sucked off and washed with a small amount of ice-cold acetone. 5.29 g (76.5 %) of the potassium salt of 2-butyl-4-chloro-1-[(2'-triphenylmethyltetrazol-5-yl)-1,1'-biphenyl-4-yl]-5-(hydroxymethyl)imidazole (III) were obtained. Mp (DSC) 229.7 °C (change of cryst. form) and 274.6 °C. ¹H NMR spectra (DMSO): 0.83 t, *J*=7.27, 3H; 1.26 m, 2H; 1.48 m, 2H; 2.51 t, *J*=7.53, 2H; 4.34 s, 2H; 5.23 s, 2H; 6.93 d, *J*=8,36, 2H; 7.13 d, *J*=8.34, 2H; 7.32-7.39 m, 3H; 7.55 m, 1H.

### Example 3

### Potassium salt of 2-butyl-4-chloro-1-[(2'-tetrazol-5-yl)-biphenyl-4-yl]-5-(hydroxymethyl)-imidazole (losartan, II)

2.10 g of calcined potassium carbonate (0.0150 mol) was added to a suspension of 10 g (0.0150 mol) of 2-butyl-4-chloro-1-[(2'-1-trityl-1*H*-tetrazol-5-yl)-1,1'-biphenyl-4-yl]-5-(hydroxymethyl)-imidazole (trityl losartan, IV) in 65 ml of anhydrous methanol and the mixture was brought to the reflux. The mixture was, after 6 hr of reflux, stirred overnight without heating. The next day the solution was concentrated to 1/3 of its volume and the after-cooling-precipitated methyltriphenylmethyl ether (XIII) was sucked off. The filtrate was evaporated and the evaporation residue crystallized after adding acetone. The crystals were sucked off and washed with a small amount of ice-cold acetone. 4.98 g (72.0 %) of the potassium salt of 2-butyl-4-chloro-1-[(2'-triphenylmethyltetrazol-5-yl)-1,1'-biphenyl-4-yl]-5-(hydroxymethyl)imidazole (III) were obtained. Mp (DSC) 233.9 °C (change of cryst. form) and 273.5 °C.

### Example 4

### Potassium salt of 2-butyl-4-chloro-1-[(2'-tetrazol-5-yl)-1,1'-biphenyl-4-yl]-5-(hydroxymethyl)-imidazole (losartan, III)

2.10 g of calcined potassium carbonate (0.0150 mol) was added to a suspension of 10 g (0.0150 mol) of 2-butyl-4-chloro-l-[(2'-1-trityl-1*H*-tetrazol-5-yl)-biphenyl-4-yl]-5-(hydroxymethyl)-imidazole (trityl losartan, IV) in 65 ml of anhydrous methanol and the mixture was brought to the reflux. The mixture was, after 5 hr of reflux, stirred overnight. The next day the solution was concentrated to 1/3 of its volume and cooled, the precipitated methyltriphenylmethyl ether (XIII) was sucked off. The filtrate was evaporated, the evaporation residue was dissolved in 30 ml of isopropylalcohol and 70 ml of cyclohexane was added to the resulting solution. The precipitated crystals were sucked off and washed with a small amount of ice-cold acetone. 5.50 g (79.5 %) of the potassium salt of 2-butyl-4-chloro-1-[(2'-triphenylmethyltetrazol-5-yl)-1,1'-biphenyl-4-yl]-5-(hydroxymethyl)imidazole (III) were obtained. Mp (DSC) 232.7 °C (change of cryst. form) and 272.9 °C.

### Example 5

### Potassium salt of 2-butyl-4-chloro-1-[(2'-1H-tetrazol-5-yl)-biphenyl-4-yl]-5-(hydroxymethyl)-imidazole (losartan, III)

1.05 g of calcined potassium carbonate (0.0075 mol) was added to a suspension of 10 g (0.015 mol) of 2-butyl-4-chloro-1-[(2'-1-trityl-1*H*-tetrazol-5-yl)-biphenyl-4-yl]-5-(hydroxymethyl)-imidazole (trityl losartan, IV) in 65 ml of anhydrous methanol and the mixture was brought to reflux in an oil bath. The heating was stopped after 8 hr and the mixture was stirred overnight. The next day the solution was concentrated to 1/3 of its volume and cooled, the precipitated methyltriphenylmethyl ether (XIIi) was sucked off. The filtrate was evaporated and the evaporation residue crystallized after adding acetone. The crystals were sucked off and washed with a small amount of ice-cold acetone. 4.98 g (72.0 %) of the potassium salt of 2-butyl-4-chloro-1-[(2'-1*H*-tetrazol-5-yl)-biphenyl-4-yl]-5-(hydroxymethyl)-imidazole (III) were obtained. Mp (DSC) 234.1 °C (change of cryst. form) and 275.2 °C.

### Example 6

### Potassium salt of 2-butyl-4-chloro-1-[(2'-1H-tetrazol-5-yl)-biphenyl-4-yl]-5-(hydroxymethyl)-imidazole (losartan, III)

1.05 g of calcined potassium carbonate (0.0075 mol) was added to a suspension of 10 g (0.015 mol) of 2-butyl-4-chloro-1-[(2'-1-trityl-1*H*-tetrazol-5-yl)-1,1'-biphenyl-4-yl]-5-(hydroxymethyl)-imidazole (trityl losartan, IV) in 65 ml of anhydrous methanol and the mixture was brought to reflux in an oil bath. The heating was stopped after 8 hr and the mixture was stirred overnight. The next day the solution was concentrated to 1/3 of its volume and cooled, the precipitated methyltriphenylmethyl ether (XIII) was sucked off. The filtrate was evaporated, the evaporation residue was dissolved in 30 ml of isopropylalcohol and 70 ml of cyclohexane was added. The precipitated crystals were sucked off and washed with a small amount of ice-cold acetone. 6.12 g (88.5 %) of the potassium salt of 2-butyl-4-chloro-1-[(2'-1*H*-tetrazol-5-yl)-biphenyl-4-yl]-5-(hydroxymethyl)-imidazole (III) were obtained. Mp (DSC) 229.1 °C (change of cryst. form) and 271.8 °C.

### Example 7

### Potassium salt of 2-butyl-4-chloro-1-[(2'-1H-tetrazol-5-yl)-biphenyl-4-yl]-5-(hydroxymethyl)-imidazole (losartan, III)

1.52 g of potassium hydrogencarbonate (0.0150 mol) was added to a suspension of 10 g (0.015 mol) of 2-butyl-4-chloro-1-[(2'-1-trityl-1*H*-tetrazol-5-yl)-1,1'-biphenyl-4-yl]-5-(hydroxymethyl)-imidazole (trityl losartan, IV) in 65 ml of anhydrous methanol and the mixture was brought to the reflux. The heating was stopped after 6 hr of the reflux and the mixture was stirred overnight. The next day the solution was concentrated to 1/3 of its volume and the after-cooling-precipitated methyltriphenylmethyl ether (XIII) was sucked off. The filtrate was evaporated and the evaporation residue crystallized after adding acetone. The crystals were sucked off and washed with a small amount of ice-cold acetone. 6.31 g (91.2 %) of the potassium salt of 2-butyl-4-chloro-1-[(2'-1*H*-tetrazol-5-yl)-biphenyl-4-yl]-5-(hydroxymethyl)-imidazole (III) were obtained. Mp (DSC) 229.9 °C (change of cryst. form) and 274.2 °C.

### Example 8

### Potassium salt of 2-butyl-4-chloro-1-[(2'-1H-tetrazol-5-yl)-biphenyl-4-yl]-5-(hydroxymethyl)-imidazole (losartan, III)

1.52 g of potassium hydrogencarbonate (0.0150 mol) was added to a suspension of 10 g (0.015 mol) of 2-butyl-4-chloro-1-[(2'-1-trityl-1*H*-tetrazol-5-yl)-1,1'-biphenyl-4-yl]-5-(hydroxymethyl)-imidazole (trityl losartan, IV) in 65 ml of anhydrous methanol and the mixture was brought to the reflux. The heating was stopped after 6 hr of the reflux and the mixture was stirred overnight. The next day the solution was concentrated to 1/3 of its volume and the after-cooling-precipitated methyltriphenylmethyl ether (XIII) was sucked off. The filtrate was evaporated and the evaporation residue crystallized after adding acetone. The crystals were sucked off and washed with a small amount of ice-cold acetone. 6.36 g (91.9 %) of the potassium salt of 2-butyl-4-chloro-1-[(2'-1*H*-tetrazol-5-yl)-biphenyl-4-yl]-5-(hydroxymethyl)-imidazole (III) were obtained. Mp (DSC) 232.9 °C (change of cryst. form) and 274.5 °C.

## Claims

1. A method for the production of a drug of formula III 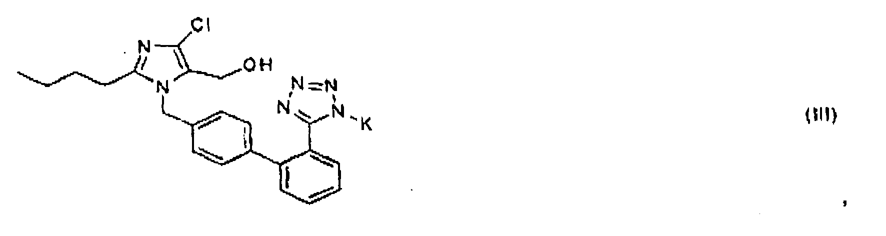 **characterized in** carrying out a solvolysis of the compound of formula IV 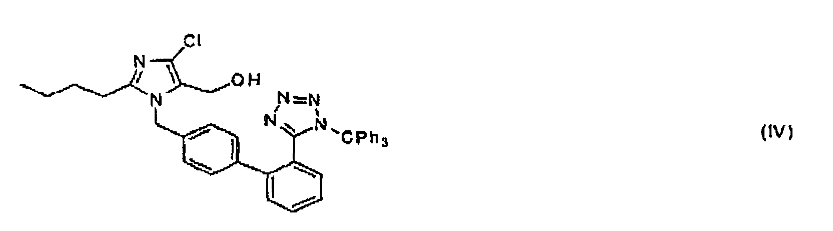 in anhydrous methanol in a neutral or slightly basic medium and a reaction with an equivalent of potassium hydrogencarbonate or potassium carbonate.

2. The method according to claim 1, wherein the solvolysis is carried out in a slightly basic medium and potassium hydrogencarbonate or potassium carbonate is added at the beginning of the reaction.

3. The method according to claim 1 or 2, wherein the product is crystallized from the mixture of an alcohol, preferably isopropanol, and an antisolvent, in which the compound of formula III is not soluble or with the use of another solvent, for example acetone.

## Patentansprüche

1. Verfahren zur Herstellung eines Arzneimittels der Formel III 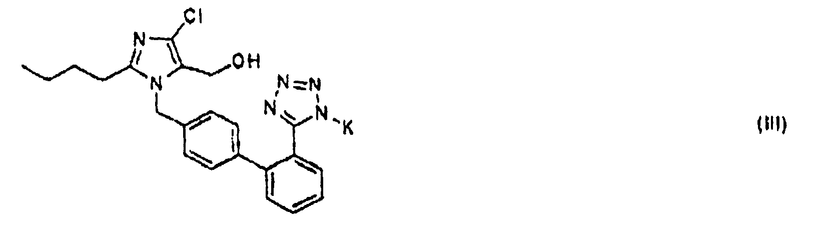 **dadurch gekennzeichnet, dass** eine Solvolyse der Verbindung der Formel IV 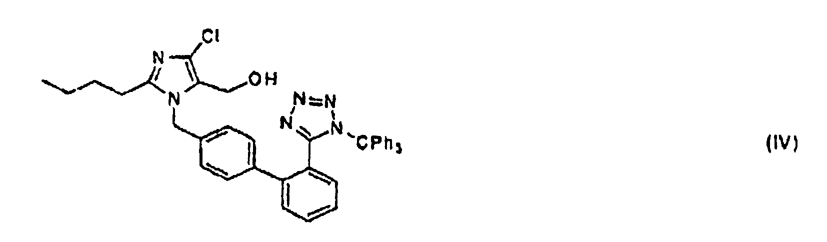 in wasserfreiem Methanol in einem neutralen oder leicht basischen Medium und eine Reaktion mit einer Äquivalente Kaliumhydrogencarbonat oder Kaliumcarbonat durchgeführt wird.

2. Verfahren gemäß Anspruch 1, wobei die Solvolyse in einem leicht basischen Medium durchgeführt wird und Kaliumhydrogencarbonat oder Kaliumcarbonat zu Beginn der Reaktion zugesetzt wird.

3. Verfahren gemäß Anspruch 1 oder 2, wobei das Produkt aus dem Gemisch eines Alkohols, vorzugsweise Isopropanol, und eines Anti-Lösungsmittels, in dem die Verbindung der Formel III nicht löslich ist, oder unter Verwendung eines anderen Lösungsmittels, z.B. Aceton, kristallisiert wird.

## Revendications

1. Un procédé pour la préparation d'un médicament de formule III 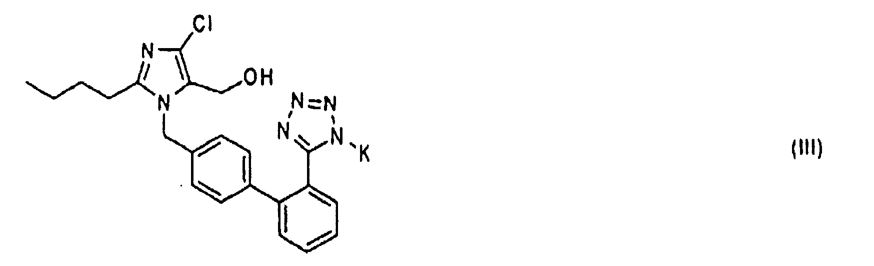 **caractérisé en ce que** l'on effectue une solvolyse de composé du formule IV 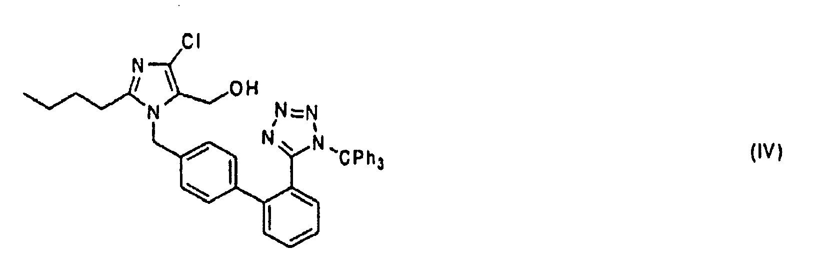 dans du méthanol anhydre, dans un milieu neutre ou légèrement basique, et une réaction avec un équivalent d'hydrogénocarbonate de potassium ou de carbonate de potassium.

2. Le procédé selon la revendication 1, dans lequel la solvolyse est effectué dans un dans un milieu légèrement basique et dans lequel l'hydrogénocarbonate de potassium ou de carbonate de potassium est ajouté au début de la réaction.

3. Le procédé selon la revendication 1 ou 2, dans lequel le produit est cristallisé dans un mélange formé d'un alcool, de préférence de l'isopropanol, et d'un antisolvant, dans lequel le composé de formule III n'est pas soluble ou avec l'utilisation d'un autre solvant, par exemple de l'acétone.
